**Europäisches Patentamt**

**European Patent Office**

**Office européen des brevets**

(19)

(11) **EP 1 186 306 A1**

(12) **EUROPEAN PATENT APPLICATION**

(43) Date of publication:
**13.03.2002 Bulletin 2002/11**

(51) Int Cl.7: **A61K 51/04**

(21) Application number: **00870188.0**

(22) Date of filing: **05.09.2000**

| | |
|---|---|
| (84) Designated Contracting States:<br>**AT BE CH CY DE DK ES FI FR GB GR IE IT LI LU MC NL PT SE**<br>Designated Extension States:<br>**AL LT LV MK RO SI**<br><br>(71) Applicant: **K.U. Leuven Research & Development**<br>**3000 Leuven (BE)**<br><br>(72) Inventors:<br>• **Lagoja, Irene**<br>**3000 Leuven (BE)** | • **Herdewijn, Piet**<br>**3111 Wezemaal (BE)**<br><br>(74) Representative:<br>**Brants, Johan Philippe Emile et al**<br>**De Clercq, Brants & Partner cv**<br>**Edgard Gevaertdreef 10a**<br>**9830 Sint-Martens-Latem (BE)** |

(54) **Labeled nucleosides and method for their preparation**

(57)    The invention relates to a method for the chemical preparation of labeled nucleosides via an oxidative-ring-opening step and a reductive-ring-closure step starting from a labeled D-glucose nucleoside resulting in the corresponding D-ribose-nucleoside by removing the carbon atom 3 on the glucose and comprising the steps of:

1. a protection of the sugar ring hydroxyl groups,
2. a sugar-base condensation, whereby the base is a purine or a pyrimidine,
3. a deprotection of the sugar ring hydroxyl groups,
4. an oxidative-ring-opening and removal of the carbon atom 3, and
5. a reductive-ring-closure resulting in labeled D-ribose-nucleoside.

The invention further relates to a compound obtainable via the method according to the invention having the formula selected from $[^{13}C,^{15}N]$ labeled 6'-O-mono- or 6'-O-bis-$C_1$-$C_6$-alkyloxytrityl-D-glycopyranosyl nucleosides, or $[^{13}C,^{15}N]$ labeled tetra-O-acetyl-D-glycopyranosyl nucleosides or $[^{13}C,^{15}N]$ labeled glycopyranosyl nucleosides.

**EP 1 186 306 A1**

**Description**

The invention relates to a method for the chemical preparation of labeled nucleosides and to the nucleotides obtainable by said method.

A bottleneck in NMR research on nucleic acids structures and dynamics is the availability of isotopically labeled materials in larger amounts and at a reasonable cost. $^{13}C/^{15}N$ labeled nucleic acids can be obtained by an enzymatic approach, in a chemical way or by a combination of both[1]. $^{13}C/^{15}N$ labeled ribomononucleotides can be isolated from bacterial cells grown in isotopically enriched media. These mononucleotides are then converted to their triphosphates using chemical or enzymatic phosphorylation. The labeled nucleoside-5'-triphosphates are then used to prepare RNA from synthetic DNA templates by *in vitro* transcription with T7 RNA polymerase[2]. The yield of labeled monomers isolated from the cultures as a mixture of four nucleotides is about 4-5%. The conversion of isotopically labeled glucose into nucleotides in a single coupled enzymatic reaction is a much more efficient process[3]. Over a gram of isotopically labeled ribomononucleotides can be obtained from 1 gram of glucose.

This method allows the large-scale preparation of uniformly isotopically labeled RNA. However, the synthesis of isotopically labeled DNA in sufficient quantity for structural analysis by NMR, is less obvious. The abundance of DNA in cells is only about 15% of that of RNA. A procedure for the enzymatic synthesis of uniformly $^{13}C$ and $^{15}N$ labeled DNA oligonucleotides has been developed only recently[4]. Enzymatic hydrolysis of DNA of bacteria grown with $^{15}N$- and $^{13}C$ enriched nutrients deliver the deoxymononucleotides, which are enzymatically phosphorylated to the triphosphates. The labeled deoxynucleotide-5'-triphosphates are incorporated into oligonucleotides using a 3'-5' exonuclease-deficient mutant of Klenow fragment of DNA polymerase I and a DNA template linked to a primer. Region-specific isotopic enrichment was also described using mutated Klenow DNA polymerase and a hairpin template[5]. This method was followed by a more efficient procedure using Taq DNA polymerase[6]. Louis et al. described a method for the large scale preparation of uniformly isotope labeled DNA by a) the growth of a suitable plasmid harboring multiple copies of the described oligonucleotide in a medium based on $^{15}N$ and $^{13}C$ nutrients or b) a polymerase chain reaction with $^{15}N$- and/or $^{13}C$ labeled deoxynucleoside triphosphates[7].

In comparison with the enzymatic methods, the chemical methods need more technical expertise[8.] The chemical synthesis, however, is much more flexible and allows the preparation of virtually all-possible isotopomeric nucleosides and site specific labeled oligomers[9]. Specific labeling will allow more accurate NMR parameters to be obtained. The chemical approach, likewise, opens the possibility to obtain labeled modified nucleosides and oligonucleotides. A more efficient and less costly chemical synthesis would have considerable advantages over the enzymatic approach.

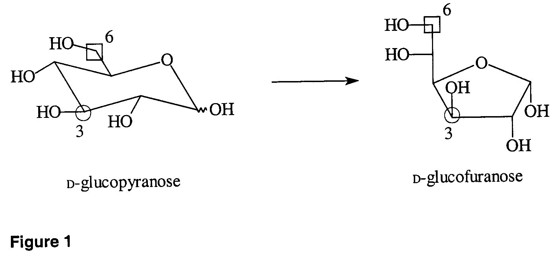

The key step in the chemical preparation of fully labeled nucleotides is the synthesis of protected $[^{13}C_5]$-D-ribose, which starts from the readily available $[^{13}C_6]$-D-glucose[10-12]. The overall yield of this conversion is 40%. The $[^{13}C_5]$-1,2,3,5-tetra-*O*-acyl-$\alpha,\beta$-D-ribofuranose is then converted into the individual protected nucleosides by Vorbrug-gen condensation[13], deprotection and tritylation reactions. The disadvantages of the published methods are the multiple steps needed to convert $[^{13}C_6]$- D-glucose into $[^{13}C_5]$- D-ribose. The reason for this is that one of the carbon atoms (C-3) of D-glucose has to be inverted in configuration while another carbon atom (C-6) has to be removed (Figure 1: Literature methods use the conversion of glucopyranose to glucofuranose to invert the configuration at C-3 and remove C-6 in an oxidative way). This is accomplished by the conversion of glucopyranose to glucofuranose. The published methods differ from each other by a) first inversion of configuration by a PDC-NaBH$_4$ reaction and then oxidative cleavage of the side chain[10]; b) first shortening of the side chain by a NalO$_4$-NaBH$_4$ reaction followed by inversion of configuration using a nucleophilic substitution reaction[11]; c) a one pot procedure to oxidative cleave the diol and reduce simultaneously both the 3-keto function and the 6-aldehyde group[12].

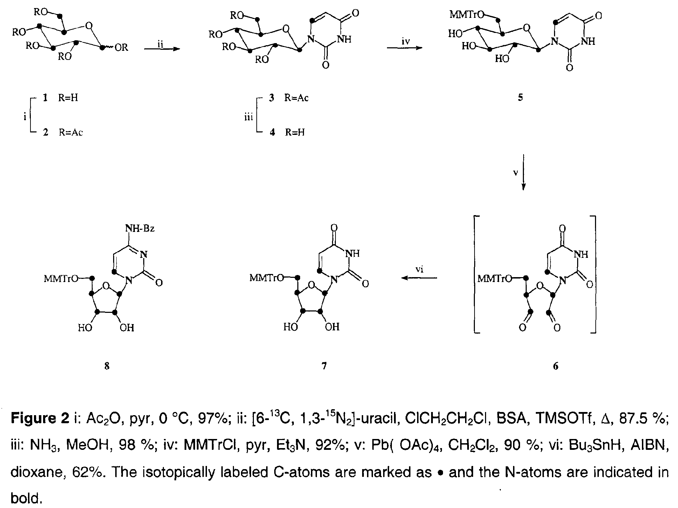

Surprisingly, an economically much more profitable approach would be the direct removal of the carbon atom with the wrong configuration i.e. carbon atom 3 of D-glucose. This can be achieved by starting with a glycopyranose nucleoside via an oxidative ring opening - reductive ring closure procedure that removes the carbon-atom 3 (Figure 2).

The invention relates therefor to a method for the chemical preparation of labeled nucleosides via an oxidative-ring-opening step and a reductive-ring-closure step starting from a labeled D-glucose nucleoside resulting in the corresponding D-ribose-nucleoside by removing the carbon atom 3 on the glucose.

In a preferred embodiment the method according to the invention starting from labeled $[^{13}C_6]$-D-glucose com-prises the steps of:

  1. a protection of the sugar ring hydroxyl groups,
  2. a sugar-base condensation, whereby the base is a purine or a pyrimidine,
  3. a deprotection of the sugar ring hydroxyl groups,
  4. an oxidative-ring-opening and removal of the carbon atom 3, and
  5. a reductive-ring-closure resulting in labeled D-ribose-nucleoside.

In a more preferred embodiment the deprotection of the sugar ring hydroxyl groups is followed by a protection step of the primairy OH-group, wherein the protection step comprises a mono-$C_1$-$C_6$-alkyloxytritylation or a bis-$C_1$-$C_6$-alkyloxytritylation of the primary hydroxyl function in the 6'-O-position of glucose.

The removed carbon atom is oxidated into formic acid. The oxidative cleavage of diols using periodate is a fast reaction. With glucopyranoses, however, the oxidative cleavage of diols is an extremely slow reaction and not useful for preparative purposes.

The reaction rate can be substantially increased by using $Pb(OAc)_4$. The feasibility of this approach has been proven by two examples in the pyrimidine series, i.e. the synthesis of [1',2',3',4',5'-$^{13}C_5$, 6-$^{13}C$, 1,3-$^{15}N_2$]-5'-O-MMTr-uridine in six reaction steps starting from D-[$^{13}C_6$]-glucose and [6-$^{13}C$, 1,3-$^{15}N_2$]-uracil in a total yield of 44% and the synthesis of [1',2',3',4',5'-$^{13}C_5$, 6-$^{13}C$, 1,3,$NH_2$-$^{15}N_3$]-5'-O-MMTr-N$^4$-benzoylcytidine using [6-$^{13}C$, 1,3-$NH_2$-$^{15}N_3$]-N$^6$-benzoylcytosine in seven steps and a total yield of 32 %. Due to its shortness and high yield, this reaction scheme can be considered as a considerable improvement over existing methods[10-12] making labeled nucleotide material more easily available.

The per acetylated [$^{13}C_6$]- D-glucose (**2**), obtained by reaction of [$^{13}C_6$]- D-glucose (**1**) with acetic anhydride in pyridine was condensed with [6-$^{13}C$, 1,3-$^{15}N_2$]uracil under Vorbruggen conditions in 87.5% yield. Full deprotection of **3** with ammonia in methanol (to **4**), followed by monomethoxytritylation of the primary hydroxyl function yielded [1',2',3',4',5',6'-$^{13}C_6$]-(6'-O-MMTr-β-D-glucopyranosyl)-[6-$^{13}C$,1,3-$^{15}N_2$]-uracil (**5**). The yield of the deacetylation reaction is 98% and of the monomethoxytritylation reaction is 92%. The glucose sugar was oxidatively cleaved using $Pb(OAc)_4$ in dichloromethane to give **6** in a yield of 90%. The reductive ring closure reaction using tri-n-butyltin hydride and AIBN in dioxane to give **7** is at present the reaction step in this scheme with the lowest yield (62%). The introduction of the monomethoxytrityl group in the 6'-O-position before carrying out the final reactions, is preferred for the stereochemical outcome of the carbon-carbon bond forming reaction and for facilitating the work-up procedure.

The feasibility of this synthetic scheme for the synthesis of the corresponding labeled cytosine nucleoside **8** has been proven. Silylated [6-$^{13}C$,1,3,$NH_2$-$^{15}N_3$]-N$^4$-benzoylcytosine was reacted with [1',2',3',4',5',6'-$^{13}C_6$]-glucose penta-O-acetate in dichloromethane in the presence of $SnCl_4$ (99% yield) after which the protecting groups were removed in basic medium (92% yield) leaving [1',2',3',4',5',6'-$^{13}C_6$]-(β-D-glucopyranosyl)- [6-$^{13}C$,1,3,$NH_2$-$^{15}N_3$]-cytosine as a crystalline material. The cytosine base was protected with a benzoyl group (90% yield) and the 6'-hydroxyl group was protected with a monomethoxytrityl group (83% yield). Reaction of [1',2',3',4',5',6'-$^{13}C_6$]-(6'-O-MMTr-β-D-glucopyranosyl)- [6-$^{13}C$,1,3,$NH_2$-$^{15}N_3$]-N$^4$-benzoylcytosine with $Pb(OAc)_4$ in dichloromethane gave the oxidized ring opened nucleoside in 92% yield. The ribose ring was obtained by reacting the dialdehyde with $Bu_3SnH$ and AIBN in dioxane in 54% yield.

In summary, we describe a new synthetic scheme leading to [$^{13}C$, $^{15}N$]-labeled 5'-O-monomethoxytrityl-uridine and 5-O-monomethoxytrityl-N$^6$-benzoylcytidine in six and seven steps respectively from D-[$^{13}C_6$]-glucose in a total yield of 44%, and 32% respectively. Given the observation that **1** can be converted into **4** in a one pot procedure and that **6** is an intermediate that does not need complete isolation, the conversion of **1** to **7** can be considered as a three-step reaction. This method has the advantage over previously described synthetic schemes because of a) its shortness (for uridine: ref. 10: 13 reactions, ref. 11: 11 reactions, ref. 12: 9 reactions), b) simple protecting group manipulation giving the monomethoxytritylated nucleoside in a straightforward way and c) a total yield which is better[10,11] than or similar[12] to existing literature procedures.

The feasibility of this synthetic scheme for the synthesis of purine nucleosides has been proven, using adenine substituted compounds as example.

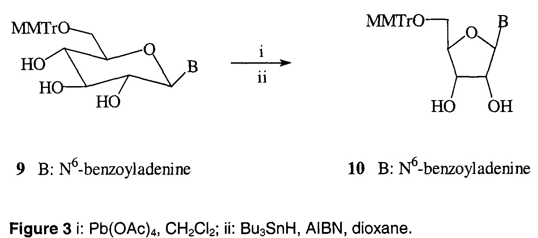

Thus, silylated N$^6$-benzoyladenine was reacted with penta-O-acetyl- D-glucose in dichloromethane using $SnCl_4$, after which the protecting groups were removed in basic medium, leaving 9-[β-D-glycopyranosyl)adenine as a crystalline material. The adenine base was protected with a benzoyl group and the 6'-hydroxyl group was protected with a monomethoxytrityl group. This gave the starting material for the two-step ring contraction reaction. Reaction of 6'-O-monomethoxytrityl-N$^6$-benzoyl-9-(β-D-glucopyranosyl)adenine with $Pb(OAc)_4$ in dichloromethane gave the oxidized material in 88%. The ribose ring was obtained by reacting the dialdehyde with $Bu_3SnH$ and AIBN in dioxane. These key reactions of the synthetic procedure, yielding 5'-O-monomethoxytrityl-N$^6$-benzoyladenosine are depicted in Figure 3 and the yields of the individual reactions are given in Table 1.

For the synthesis of the guanine nucleoside, a transglycosylation reaction starting from tri-O-acetyl-uridine is preferred. Therefore a mixture of tri-O-acetyl-uridine and N$^2$-isobutyrylguanine are treated respectively with BSA and trimethylsilyl triflate under reflux.

As an alternative to tin hydride, the reductive ring closure can likewise be carried out with $Sm(II)I_2$. This method is used for the stereocontrolled formation of cyclic vicinal diols. The first examples of such intramolecular pinacol couplings using transition metal catalysts have been described by Kagan et al. and Molander and Kenny. The following procedure is given only as an example of alternative ring closure procedures.

In a typical procedure a solution of $SmI_2$ in THF (0.1 M, 10.8 mL) was cooled to -78°C. Under nitrogen atmosphere a mixture of the Uracil-dialdehyde (0.222g, 0.431 mmol) in THF (10 mL) and THF/t-BuOH (1.1 mL, 1 M) was added over a period of 15 min. After stirring the reaction mixture at -78°C for one hour the temperature was slowly increased to room temperature over a period of 2 hours. After addition of sodium bicarbonate (50 mL) the white slurry was extracted with ethyl acetate (5 x 60 mL). The organic phase was washed with 10 % $Na_2S_2O_3$ (50 mL), dried over $Na_2SO_4$, and after the solvent was removed, the residue was purified by column chromatography (silica, 15 x 2 cm, $CH_2Cl_2$ (80 ml), followed by a gradient from $CH_2Cl_2$ to $CH_2Cl_2/MeOH$ = 95 : 5). MMT-Uridine was obtained in 64 % yield slightly contaminated with enantiomeric impurities, which were removed by HPLC.

To obtain deoxyribonucleosides selectively, the ribonucleosides can be deoxygenated at the 2'-position according to Robins et al. 5'-O-Dimethoxytritylation and phosphitylation lead to the DNA phosphoramidites.

Table 1:

| Yield of the reactions used for the synthesis of the protected ribose nucleosides | | | |
|---|---|---|---|
| | [6-$^{13}$C,1,3-$^{15}$N$_2$]-uracil | adenine | [6-$^{13}$C,1,3,NH$_2$-$^{15}$N$_3$]-cytosine |
| Sugar-base condensation reaction* | 87.5 % | 74 % | 94 % |
| Full deprotection of the nucleosides | 98 % | 94 % | 92 % |
| Protection of the base moieties ** | - | 75 % | 90 % |
| Monomethoxytritylation | 92 % | 90 % | 83 % |
| Oxidative ring opening | 90 % | 88 % | 92 % |
| Reductive ring closure | 62 % | 53 % | 54 % |

* reactions are carried out with N[6]-benzoyladenine and N[4]-benzoylcytosine

** as well the adenine as cytosine base are protected with a benzoyl group using the transient protection procedure

EXPERIMENTAL

NMR spectra were recorded on a Varian, Gemini 200 spectrometer ($^1$H-200 MHz, $^{13}$C 50 MHz). All NH/OH protons were assigned by exchange with $D_2O$. Mass spectra were recorded on a Kratos Concept 1H mass spectrometer (Ms, LSIMS [m/z] (%)); Samples were dissolved in glycerol (Gly) / thioglycerol (Thgly) / m-nitrobenzyl alcohol (NBA) and the secondary ions were accelerated at 6 kV. Scans were performed at 10 sec / decade). Exact mass measurements were performed on a quadrupole - time of flight mass spectrometer (Q-Tof-2, Micromass, Manchester, UK) equipped with a standard electrospray ionization (ESI)interface. Samples were infused in a 2-propanol : water (1 : 1) mixture at 3 mL/min. TLC was performed with TLC aluminum sheets (Merck, Silica gel 60 $F_{254}$) and silica (200 - 425 mesh) was used for column chromatography. Melting points (mp [°C]) were determined with a Büchi-SMP - 20 capillary melting point apparatus. For all reactions dry (molecular sieve) analytical grade solvents were used. Solvents for column chromatography were used without any further purification.

PREPARATION OF FULLY LABELED GLUCOSE PENTA-*O*-ACETATE:

After cooling pyridine (20 mL) to 0 C, acetic anhydride (15 g, 14 mL, 0.15 mmol) was added. The fully labeled glucose (3.0 g, 0.016 mmol) was added in small portions at 0 C. The reaction temperature was slowly allowed to come to ambient temperature and stirred at room temperature overnight. After addition of water (60 mL) an oil is formed, which turns solid on scrapping. The resulting precipitate is filtered off and recrystallized from water. The ratio of α : β enantiomer is 1 : 1 ($^{13}$C NMR).

[1,2,3,4,5,6-$^{13}$C$_6$]-Glucose penta-*O*-acetate: Yield: 6.20 g (97 %) $R_f$ ($CH_2Cl_2/MeOH$ = 95 : 5): 0.63; mp: 110 - 112 C ($H_2O$); $^1$H-NMR (200 MHz, $CDCl_3$):δ = 2.01 - 2.18 (5 x 3H Ac), 3.74 - 4.81 (4H, m, 2 x H-6', H5', H3'), 5.44-5.56 (1H, m, H4'), 5.28, 6.13 (1H, d x d, $J^1$ = 167 Hz, $J^2$ = 8.6 Hz; H1' β-isomer), 5.88, 6.76 (1H, d x m, $J^1$ = 167 Hz; H1' α-isomer); $^{13}$C-NMR (50 MHz, $CDCl_3$): δ = 20.2-20.5 (5 x $CH_3$ Ac), 169.5 - 170.7 (5 x C=O Ac), 60.9, 61.8 (d, $J$= 44 Hz, $CH_2$-6'), 67.2 - 71.0 (m, 3 CH, CH-4',2',3'), 72.7 (t, $J$= 44 Hz, CH-5'), 88.0, 88,6 (d, $J$= 44 Hz, CH-1'α), 91.4, 92.0 (d, $J$= 44 Hz, CH-1'β); MS (LSIMS; Thgly; m/z (%)): 337 (73.3) [M + H]$^+$.

GLYCOSYLATION REACTION OF URACIL WITH FULLY LABELED GLUCOSE PENTA-*O*-ACETATE:

To a suspension of [6-13C,1,3-$^{15}$N$_2$]-uracil (0.96 g, 8.32 mmol) and [$^{13}$C$_6$]-glucosepenta-*O*-acetate (3.3 g, 8.32 mmol) in dichloroethane (40 mL) BSA (4.8 mL, 20 mmol) was added. The mixture was stirred under nitrogen at ambient temperature for 20 min to give a clear, colorless solution. After addition of TMSOTf (3.60 mL, 20 mmol) under nitrogen the reaction mixture was heated under reflux for 2 h. After cooling to ambient temperature the resultant brown mixture was evaporated in *vacuo*. The resulted oil was diluted in ethyl acetate (200 mL) and washed with NaHCO$_3$ (150 mL) and brine NaCl-solution (2 x 100 mL). After drying over $Na_2SO_4$ and evaporating the solvent, the resultant oil was purified by column chromatography (silica, 24 x 3 cm, ethyl acetate/petroleum ether = 2 : 1).

[1',2',3',4',5',6'-$^{13}C_6$]-(2',3',4',6'-Tetra-*O*-acetyl-β-D-glucopyranosyl)-uracil: Yield: 3.17 g (85 %); $R_f$ (ethyl acetate/petrol ether = 2 : 1): 0.34; mp: 152 -154 °C (EtOH°); $^1$H-NMR (500 MHz, CDCl$_3$):δ = 2.00 - 2.09 (4 x 3H, 4 x CH$_3$ Ac), 3.92 (1H, $J^{CH}$ = 162 Hz, H5'), 4.11 - 4.29 (2H, $J^{CH}$ = 162 Hz, 2 x H6'), 5.15 (2H, $J^{CH}$ = 162 Hz, H3', H4'), 5.38 (1H, $J^{CH}$ = 162 Hz, $J^{HH}$ = 5 Hz, H2'), 5.81 (1H, d, $J$ = 8.2 Hz, H-5), 5.85 (1H, $J^{CH}$ = 162 Hz, $J^{HH}$ = 9 Hz, H1'), 7.33 (1H, d, $J$ = 8.2 Hz, 6-H), 9.51 (1H, ex, s, br, NH); $^{13}$C-NMR (125.6 MHz, CDCl$_3$): δ = 20.2, 20.3, 20.4, 20.6 (4 x CH$_3$ Ac), 61.5 (d, $J$= 43 Hz, CH6'), 67.8 (t, $J$= 43 Hz, CH4'), 69.3 (t, $J$= 43 Hz, CH2'), 72.7 (t, $J$= 43 Hz, CH3'), 75.0 (t, $J$= 43 Hz, CH5'), 80.0 (d, $J$ = 43 Hz, CH1'), 103.8 (s, 5-CH), 139.1 (s, 6-CH), 150.5 (s, 2-C), 162.7 (s, 4-C), 169.5, 169.6, 169.8, 170.5 (4 x C=O Ac); MS (LSIMS; Thgly; m/z (%)): 115 (55), 175 (100), 337 (48) [Gluc + H]$^+$, 449 (19) [M + H]$^+$; Exact mass: $C_{12}C_6^*H_{23}N_2O_{11}$: Calc.: 449.1499; Found: 449.1458.

[1',2',3',4',5',6'-$^{13}C_6$]-(2',3',4',6'-Tetraacetyl-β-D-glucopyranosyl)-[6-$^{13}$C,1,3-$^{15}N_2$]-uracil: Yield: 3.29 g (87.5 %); $R_f$ (ethyl acetate/petrol ether = 2 : 1 ): 0.34; mp: 153 - 155 °C (EtOH); $^1$H-NMR (500 MHz, CDCl$_3$): δ = 2.00 - 2.09 (4 x 3H, 4 x CH$_3$ Ac), 3.92 (1H, $J^{CH}$ = 162 Hz, H5'), 4.11 - 4.29 (2H, $J^{CH}$ = 162 Hz, 2 x H6'), 5.15 (2H, $J^{CH}$ = 162 Hz, H3', H4'), 5.38 (1H, $J^{CH}$ = 162 Hz, $J^{HH}$ = 5 Hz, H2'), 5.81 (1H, m, H-5), 5.85 (1H, $J^{CH}$ = 162 Hz, $J^{HH}$ = 9 Hz, H1'), 7.36 (1H, $J^{CH}$ = 182 Hz, $J^{HH}$ = 8 Hz, 6-H), 9.11, (1H, ex, d, br, $J$ = 90 Hz, NH); $^{13}$C-NMR (125.6 MHz, CDCl$_3$): □= 20.2, 20.3, 20.4, 20.6 (4 x CH$_3$ Ac), 61.5 (d, $J$= 43 Hz, CH6'), 67.8 (t, $J$= 43 Hz, CH4'), 69.3 (t, $J$= 43 Hz, CH2'), 72.7 (t, $J$= 43 Hz, CH3'), 75.0 (t, $J$= 43 Hz, CH5'), 80.3 (d x d, $J^1$ = 14.6 Hz, $J^2$ = 45 Hz, CH1'), 103.7 (d x d, $J^1$= 14.6 Hz, $J^2$= 66 Hz, 5-CH), 139.2 (d, $J$= 14.6 Hz, 6-CH), 150.5 ((t), $J$ = 11 Hz, 2-C), 162.9 (d, $J$ = 11 Hz, 4-C), 169.5, 169.6, 169.8, 170.5 (4 x C=O Ac); MS (LSIMS; Thgly; m/z (%)): 115 (62), 175 (100), 337 (30) [Gluc + H]$^+$, 452 (6) [M + H]$^+$; Exact Mass: $C_{11}C_7^*H_{23}N_2^*O_{11}$: Calc.: 452.1473; Found: 452.1510.

<u>FULL DEPROTECTION - GENERAL PROCEDURE:</u>

A solution of the tetra-*O*-acetyl protected nucleoside U (10 mmol) in NH$_3$ / MeOH (20 mL) was stirred overnight at ambient temperature. After removal of the solvent in *vacuo* the precipitate was recrystallized from dichloromethane.

[1',2',3',4',5',6'-$^{13}C_6$]-(β-D-Glucopyranosyl)-uracil: Yield: 2.69 9(96 %) $R_f$ (ethyl acetate): 0.1; mp: 201□C (MeOH); $^1$H-NMR (500 MHz, DMSO-d$_6$):δ = 3.14 (1H, t, $J^{CH}$ = 162 Hz, $J^{HH}$ = 5 Hz, H4'), 3.25 - 3.32 (2H, $J^{CH}$ = 162 Hz, H2', H3'), 3.35 - 3.45 (2H, $J^{CH}$ = 162 Hz, H5', H6'), 3.65 (1H, $J^{CH}$ = 162 Hz, H6'), 5.29 (1H, $J^{CH}$ = 160 Hz, $J^{HH}$ = 9 Hz, H1'), 5.62 (1H, d, $J^{HH}$ = 8 Hz, H-5), 7.64 (1H, d, $J^{HH}$ = 8 Hz, 6-H), 4.04 (1H, d, br, ex, J = 5.2 Hz, OH), 4.52, (1H, t, br, ex, $J$ = 5.6 Hz, 6'-OH), 5.01 (1H, d, br, ex, $J$ = 5.2 Hz, OH), 5.14, (1H, d, br, ex, $J$ = 5.2 Hz, OH), 10.99 (1H, s, br, ex, NH); $^{13}$C-NMR (125.6 MHz, DMSO-d$_6$): □ = 60.9 (d, $J$= 43 Hz, CH6'), 69.6 (t, $J$= 43 Hz, CH4'), 70.8 (t, $J$= 43 Hz, CH2'), 76.9 (t, $J$= 43 Hz, CH3'), 79.9 (t, $J$= 43 Hz, CH5'), 82.5 (d x d, $J^1$ = 14.6 Hz, $J^2$ = 45 Hz, CH1'), 102.1 (s, 5-CH), 141.7 (s, 6-CH), 151.2 (s, 2-C), 163.3 (s, 4-C); MS (LSIMS; Gly,TFA; m/z (%)): 91 (100), 281 (9) [M + H]$^+$; Exact Mass: $C_4C_6^*H_{15}N_2O_7$: Calc.: 281.1081; Found: 281.1094.

[1',2',3',4',5',6'-$^{13}C_6$]-(β-D-Glucopyranosyl)-[6-$^{13}$C, 1,3-$^{15}N_2$]-uracil: Yield: 2.77 9(98 %) $R_f$ (ethyl acetate): 0.1; mp: 202□C (MeOH); $^1$H-NMR (500 MHz, DMSO-d$_6$):δ = 3.14 (1H, t, $J^{CH}$ = 162 Hz, $J^{HH}$ = 5 Hz, H4'), 3.25 - 3.32 (2H, $J^{CH}$ = 162 Hz, H2', H3'), 3.35 - 3.45 (2H, $J^{CH}$ = 162 Hz, H5', H6'), 3.65 (1H, $J^{CH}$ = 162 Hz, H6'), 5.29 (1H, $J^{CH}$ = 160 Hz, $J^{HH}$ = 9 Hz, H1'), 5.61 (1H, m, H-5), 7.60 (1H, $J^{CH}$ = 182 Hz, $J^{HH}$ = 8 Hz, 6-H), 4.04 (1H, d, br, ex, $J$ = 5.2 Hz, OH), 4.52, (1H, t, br, ex, $J$= 5.6 Hz, 6'-OH), 5.01 (1H, d, br, ex, $J$ = 5.2 Hz, OH), 5.14 (1H, d, br, ex, $J$ = 5.2 Hz, OH), 11.4 (1H, d, br, ex J = 90 Hz, NH); $^{13}$C-NMR (125.6 MHz, DMSO-d$_6$): δ = 60.9 (d, $J$= 43 Hz, CH6'), 69.6 (t, $J$= 43 Hz, CH4'), 70.8 (t, $J$= 43 Hz, CH2'), 76.9 (t, $J$= 43 Hz, CH3'), 79.9 (t, $J$= 43 Hz, CH5'), 82.5 (d x d, $J^1$ = 14.6 Hz, $J^2$ = 45 Hz, CH1'), 101.3 (d x d, $J^1$= 14.6 Hz, $J^2$= 66 Hz, 5-CH), 141.0 (d, $J$= 14.6 Hz, 6-CH), 151.0 ((t), $J$ = 11 Hz, 2-C), 163.3 (d, $J$ = 11 Hz, 4-C); MS (LSIMS; Gly,TFA; m/z (%)): 284 (5) [M + H]$^+$; Exact Mass: $C_3C_7^*H_{30}N_2^*O_8$: Calc.: 284.1055; Found: 284.1061.

<u>INTRODUCTION OF MONOMETHOXYTRITYL IN THE 6' POSITION: GENERAL PROCEDURE:</u>

The mixture of nucleoside U (3.5 mmol), and MMTCI (2.16 g, 7.0 mmol) in pyridine (20 mL) and triethylamine (5 mL) was stirred in an inert atmosphere at ambient temperature for 18 h. After removing the solvent in vacuo the resulted oil was coevaporated with toluene (4 x 2 mL). The resultant foam was purified by column chromatography. (silica, 15 x 3 cm, CH$_2$Cl$_2$/MeOH = 9 : 1).

[1',2',3',4',5',6'-$^{13}C_6$]-6'-*O*-Monomethoxytrityl-(β-D-glucopyranosyl)-uracil: Yield: 1.68 g (85 %); $R_f$ (CH$_2$Cl$_2$/MeOH = 9 : 1): 0.31; mp: 168 - 170°C; $^1$H-NMR (500 MHz, DMSO-d$_6$):δ = 3.63 (3H, s, CH$_3$O), 3.00 - 3.05 (1H, m, $J^{CH}$ = 162 Hz, H5'), 3.18 - 3.30 (2H, m, $J^{CH}$ = 162 Hz, 2 x H6'), 3.45 (1H, (t), $J^{CH}$ = 162 Hz, $J^{HH}$ = 5Hz, H4'), 3.53 (1H, (t), $J^{CH}$ = 162 Hz, $J^{HH}$ = 5 Hz, H3'), 5.38 (1H, $J^{CH}$ = 160 Hz, $J^{HH}$ = 9 Hz, H1'), 5.72 (1H, d, $J$ = 8.2 Hz, H-5), 5.05 (1H, br, ex, OH), 5.28, (1H, br, ex, OH), 5.40 (1H, br, ex, OH), 6.87 - 7.73 (m, 15 H, H-Ar MMT + 6-H), 11.4 (1H, s, br, ex, NH); $^{13}$C-NMR (125.6 MHz, DMSO-d$_6$): δ = 55.1 (s, CH$_3$O), 63.7 (d, $J$= 43 Hz, CH6'), 69.6 (t, $J$= 43 Hz, CH4'), 70.6 (t, $J$= 43 Hz, CH2'), 76.6 (t, $J$= 43 Hz, CH3'), 77.9 (t, $J$= 43 Hz, CH5'), 82.4 (d, $J$ = 45 Hz, CH1'), 85.5 (C, MMT), 102.0 (s, 5-CH), 113.2 (s, 2CH, AA'BB', MMT), 126.9-130.3 (CAr, MMT), 135.4 (s, 1C, AA'BB', MMT), 141.4 (s, 6-CH), 144.7 (s, 2 x 1C, MMT), 151.0 (s, 2-C), 158.3 (s, 4C, AA'BB', MMT), 163.2 (s, 4-C); MS (LSIMS; Thgly; m/z): 273 (100) [MMT]$^+$, 575 (10) [M + Na]$^+$ 597 (16) [M-H + 2Na]$^+$; Exact Mass: C$_{24}$C$_6^*$H$_{30}$N$_2$O$_8$Na: Calc.: 575.2102; Found: 575.2116

[1',2',3',4',5',6'-$^{13}$C$_6$]-6'-*O*-Monomethoxytrityl-(β-D-glucopyranosyl)-[6-$^{13}$C,1,3-$^{15}$N$_2$]-uracil: Yield: 1.8 g (92 %); R$_f$ (CH$_2$Cl$_2$/MeOH = 9 : 1): 0.31; mp: 168 - 170°C; $^1$H-NMR (500 MHz, DMSO-d$_6$):δ = 3.63 (3H, s, CH$_3$O), 3.00 - 3.05 (1H, m, $J^{CH}$ = 162 Hz, H5'), 3.18 - 3.30 (2H, m, $J^{CH}$ = 162 Hz, 2 x H6'), 3.45 (1H, (t), $J^{CH}$ = 162 Hz, $J^{HH}$ = 5Hz, H4'), 3.53 (1H, (t), $J^{CH}$ = 162 Hz, $J^{HH}$ = 5 Hz, H3'), 5.38 (1H, $J^{CH}$ = 160 Hz, $J^{HH}$ = 9 Hz, H1'), 5.72 (1H, m, H-5), 5.04 (1H, br, ex, OH), 5.26, (1H, br, ex, OH), 5.41 (1H, br, ex, OH), 6.87 - 7.42 (m, 15 H, H-Ar MMT + 6-H), 11.4 (1H, ex, d, br, $J$ = 90 Hz, NH); $^{13}$C-NMR (125.6 MHz, DMSO-d$_6$): δ = 55.1 (s, CH$_3$O), 63.7 (d, $J$= 43 Hz, CH6'), 69.6 (t, $J$= 43 Hz, CH4'), 70.6 (t, $J$= 43 Hz, CH2'), 76.6 (t, $J$= 43 Hz, CH3'), 77.9 (t, $J$= 43 Hz, CH5'), 82.3, 82.6 (d x d, $J^1$ = 14.6 Hz, $J^2$ = 45 Hz, CH1'), 85.8 (C, MMT), 101.3 (d x d, $J^1$= 14.6 Hz, $J^2$= 66 Hz, 5-CH), 113.3 (s, 2CH, AA'BB', MMT), 126.9-130.3 (CAr, MMT), 135.4 (s, 1C, AA'BB', MMT), 141.4 (d, $J$= 14.6 Hz, 6-CH), 144.7 (s, 2 x 1C, MMT), 151.0 ((t), $J$ = 11 Hz, 2-C), 158.3 (s, 4C, AA'BB', MMT), 163.3 (d, $J$= 11 Hz, 4-C); MS (LSIMS; Thgly; m/z): 273 (100) [MMT]$^+$, 578 (2) [M + Na]$^+$ 600 (12) [M - H + 2Na]$^+$; Exact Mass: C$_{23}$C$_7^*$H$_{29}$N$_2^*$O$_8$Na$_2$: Calc.: 600.1895; Found: 600.1864.

OXIDATIVE RING OPENING OF U: GENERAL PROCEDURE:

To a solution of 6'*O*-MMT nucleoside U (1 mmol) in absolute dichloromethane (10 mL), Pb(OAc)$_4$ (1.33 g, 3 mmol) is added under nitrogen at ambient temperature. The solution gets warm and turns yellow. After 2 h a colorless precipitate of Pb(OAc)$_2$ is formed. Stirring is continued overnight. After removing the solvent on a rotavapor the residue is purified by column chromatography (silica, 10 x 2 cm, CH$_2$Cl$_2$/MeOH = 95 : 5). MS verified the structure of the resulted colorless foams because of the complicated equilibrium observed in the NMR[14].

[1',2',3',4',5'-$^{13}$C$_5$]-5'-*O*-Monomethoxytrityl-uridine dialdehyde: Yield: 0.43 g (89 %); R$_f$ (CH$_2$Cl$_2$/MeOH = 9 : 1) 0.44, MS (ESI; m/z (%)): 273 (100) [MMT]$^+$, 542 (2) [M + Na]$^+$; Exact Mass: C$_{24}$C$_5^*$H$_{26}$N$_2$O$_8$Na: Calc.: 542.1802; Found: 545.1813.

[1',2',3',4',5'-$^{13}$C$_5$]-5'-*O*-Monomethoxytrityl-[6-$^{13}$C, 1,3-$^{15}$N$_2$]-uridine dialdehyde: Yield: 0.49 g (90 %); R$_f$ (CH$_2$Cl$_2$/MeOH = 9 : 1) 0.44, MS (ESI; m/z (%)): 273 (100) [MMT]$^+$, 545 (2) [M + Na]$^+$; Exact Mass: C$_{23}$C$_6^*$H$_{26}$N$_2^*$O$_8$Na: Calc.: 545.1774; Found: 545.1766.

REDUCTIVE RING CLOSURE OF U: GENERAL PROCEDURE:

U-dialdehyde (1 mmol), was dissolved in absolute dioxane (8 mL) under a nitrogen atmosphere. Tri-n-butyltin hydride (0.43 g, 0.4 mL, 1.25 mmol) is added *via* a septum. The reaction mixture is heated to 90°C. Via the septum a solution of AIBN (0.02 g, 0.12 mmol) in dioxane (2 mL) is added. Foam is formed, after stirring for 30 min at 90°C the reaction mixture was checked with TLC (CH$_2$Cl$_2$/MeOH = 95 : 5). If unreacted aldehyde is monitored on TLC, some more AIBN (0.01g, 0.06 mmol) and tri-n-butyltin hydride (0.11g, 0.1 mL, 0.31 mmol) are added and stirring at 90°C is continued for another 60 min. After evaporating the solvent under reduced pressure the residue is purified by column chromatography (silica, 20 x 3 cm, CH$_2$Cl$_2$ (100 mL) → CH$_2$Cl$_2$/MeOH = 95 : 5).

[1',2',3',4',5'-$^{13}$C$_5$]-5'-Monomethoxytrityl-uridine: 0.34 g, (58 %); R$_f$ (CH$_2$Cl$_2$/MeOH =95 : 5): 0.41; mp: 100 - 105°C (CH$_2$Cl$_2$); $^1$H-NMR (500 MHz, DMSO-d$_6$):δ = 3.56 (3H, s, CH$_3$O), 3.00 - 3.05 (2H, m, $J^{CH}$ = 162 Hz, 2x H5'), 4.00 (1H, m, $J^{CH}$ = 162 Hz, H2'), 4.13 (2H, m, $J^{CH}$ = 162 Hz, H3', H4'), 5.16 (1H, br, ex, OH), 5.30 (1H, d, $J$ = 8 Hz, H-5), 5.51, (1H, br, ex, OH), 5.80 (1H, $J^{CH}$ = 160 Hz, $J^{HH}$ = 6.4 Hz, H1'), 6.89 - 7.42 (m, 15 H, H-Ar MMT + 6-H), 11.37 (1H, s, br, ex, NH); $^{13}$C-NMR (50 MHz, DMSO-d6): δ = 55.2 (s, CH$_3$O), 63.0 (d, $J$= 43 Hz, CH5'), 69.6 (t, $J$= 43 Hz, CH2'), 73.6 (t, $J$= 43 Hz, CH3'), 82.5 (t, $J$= 43 Hz, CH4'), 89.0 (d, $J$ = 45 Hz, CH1'), 86.3 (C, MMT), 101.6 (s, 5-CH), 113.5 (s, 2CH, AA'BB', MMT), 124.1-130.3 (CAr, MMT), 134.8 (s, 1C, AA'BB', MMT), 140.8 (s, 6-CH), 144.1, 144.4 (s, 2 x 1C, MMT), 150.7 (s, 2-C), 158.5 (s, 4C, AA'BB', MMT), 163.3 (s, 4-C); MS (ESI; m/z (%)): 273 (100) [MMT]$^+$, 544 (34) [M + Na]$^+$; Exact Mass: C$_{24}$C$_5^*$H$_{28}$N$_2$O$_7$Na: Calc.: 544.1959; Found: 544.1962.

[1',2',3',4',5'-$^{13}$C$_5$]-5'-Monomethoxytrityl-[6-$^{13}$C, 1,3-$^{15}$N$_2$]-uridine: 0.34 g, (57 %); R$_f$ (CH$_2$Cl$_2$ : MeOH =95 : 5): 0.41; mp: 100 - 105°C (CH$_2$Cl$_2$); $^1$H-NMR (500 MHz, DMSO-d$_6$): δ = 3.56 (3H, s, CH$_3$O), 3.00 - 3.05 (2H, m, $J^{CH}$ = 162 Hz, 2x H5'), 4.00 (1H, m, $J^{CH}$ = 162 Hz, H2'), 4.13 (2H, m, $J^{CH}$ = 162 Hz, H3', H4'), 5.16 (1H, br, ex, OH), 5.30 (1H, m, H-5), 5.51, (1H, br, ex, OH), 5.80 (1H, $J^{CH}$ = 160 Hz, $J^{HH}$ = 6.4 Hz, H1'), 6.89 - 7.42 (m, 15 H, H-Ar MMT + 6-H), 11.37 (1 H, d, br, ex, $J$ = 90 Hz, NH); $^{13}$C-NMR (50 MHz, DMSO-d$_6$): δ = 55.2 (s, CH$_3$O), 63.0 (d, $J$= 43 Hz, CH5'), 69.6 (t, $J$= 43 Hz, CH2'), 73.6 (t, $J$= 43 Hz, CH3'), 82.5 (t, J= 43 Hz, CH4'), 89.0 (d x d, $J^1$ = 12 Hz, $J^2$ = 45 Hz, CH1'), 86.3 (C, MMT), 101.6 (d x d, $J^1$= 14.6 Hz, $J^2$= 66 Hz, 5-CH), 113.5 (s, 2CH, AA'BB', MMT), 124.1-130.3 (CAr, MMT), 134.8 (s, 1C, AA'BB', MMT), 140.8 (d, $J$= 14.6 Hz, 6-CH), 144.1, 144.4 (s, 2 x 1C, MMT), 150.7 ((t), $J$ = 11 Hz, 2-C), 158.5 (s, 4C, AA'BB', MMT), 163.3 (d, $J$ = 11 Hz, 4-C); MS (ESI; m/z (%)): 273 (100) [MMT]$^+$, 547 (34) [M + Na]$^+$; Exact Mass: C$_{23}$C$_6^*$H$_{28}$N$_2^*$O$_7$Na: Calc.: 547.1930; Found: 547.1957.

GLYCOSYLATION REACTION OF ADENINE AND CYTOSINE:

To the suspension of N[4]-benzoylcytosine (2.16 g, 10 mmol) or N[6]-benzoyladenine (2.40 g, 10 mmol), respectively and glucose penta-*O*-acetate (4.13 g, 10.6 mmol) in dichloromethane (40 mL) BSA (4.90 mL, 20 mmol) was added. The mixture was stirred under nitrogen at ambient temperature for 20 min resulting in a clear, colorless solution. After addition of $SnCl_4$ (3.52 mL, 30 mmol) under nitrogen the reaction mixture was heated under reflux for 2 h. The resultant brown mixture was cooled to ambient temperature, and after removal of the solvent in *vacuo* the resulting oil was diluted in ethyl acetate (200 mL) and washed with $NaHCO_3$ (150 mL). The voluminous precipitate of $Sn(OH)_4$ was filtered off and washed with ethyl acetate (5 x 50 mL). The combined organic layers were washed with brine (2 x 100 mL) and dried over $Na_2SO_4$. After evaporating the solvent the residual oil was purified by column chromatography (silica, 24 x 3 cm, ethyl acetate/petrol ether =2:1).
N[6]-Benzoyl-9-(2',3',4',6'-tetraacetyl-β-D-glucopyranosyl)adenine: Yield: 4.25 g (74 %); $R_f$ (ethyl acetate): 0.47; mp: 168-169°C (methanol; lit: 171°C, ethanol)[15]
N[4]-Benzoyl-(2',3',4',6'-tetraacetyl-β-D-glucopyranosyl)cytosine: Yield: 7.2 g (94 %); $R_f$ (ethyl acetate): 0.48; mp: 258-260°C (methanol; lit: 260°C)[15]

FULL DEPROTECTION - GENERAL PROCEDURE:

A solution of the tetra-*O*-acetyl protected nucleoside A, C (10 mmol) in $NH_3$ / MeOH (20 mL) was stirred overnight at ambient temperature. After removal of the solvent in *vacuo* the precipitate was recrystallized from dichloromethane.
9-(β-D-Glucopyranosyl)adenine: Yield: 3.26 g (94 %); $R_f$ (ethyl acetate) 0.1, mp: 200-203°C ($CH_2Cl_2$, lit: 204-206°C)[16].
1-(β-D-Glucopyranosyl)cytosine : Yield: 2.50 g (92 %); $R_f$ (ethyl acetate) 0.1, mp: 195-196°C ($CH_2Cl_2$, lit: 197 - 199°C)[17].

N-BENZOYLATION OF A AND C *VIA* TRANSIENT PROTECTION: GENERAL PROCEDURE:

In an inert atmosphere, TMSCl (1.80 g, 2 mL, 16 mmol) was added to a solution of 9-(β-D-glucopyranosyl) adenine (0.95 g, 3.2 mmol) or (β-D-glucopyranosyl)cytosine (0.85 g, 3.2 mmol), respectively, in pyridine (8 mL). The mixture was stirred at ambient temperature for 15 min. After addition of benzoyl chloride (0.91 g, 0.76 mL, 6.4 mmol) stirring was continued for another 3 h. The reaction mixture was cooled to 0°C, ice water (10 mL) was added and after stirring for 15 min an addition of comp, aqueous $NH_3$. (20 mL) followed. The solvent was removed in *vacuo* after stirring for another 30 min at ambient temperature. The resultant oil was diluted with ethyl acetate (100 mL) and washed with water (20 mL). After drying the organic layer over $Na_2SO_4$ the solvent was evaporated and the resultant oil was treated with MeOH /water (1 : 1, 14 mL) and ammonium chloride (2 g). Afterwards the mixture was evaporated to dryness and purified by column chromatography (silica, 24 x 3 cm, $CH_2Cl_2$/MeOH = 9 : 1 ).
N[6]-Benzoyl-9-(β-D-glucopyranosyl)adenine: Yield: 0.96 g (75%); $R_f$ ($CH_2Cl_2$/MeOH = 8 : 2) 0.5, mp: 192 - 196°C; $^1$H-NMR (200 MHz, DMSO-$d_6$): δ = 3.39 - 3.48 (4H, m, 2 x H6', H5', H3'), 3.69 (1H, m, (t), H4'), 4.11 (1H, m, (t), H2'), 4.65, 5.22, 5.35, 5.45 (4H, ex, 4 x OH), 5.58 (1H, d, *J* = 9 Hz, H1'), 7.51 - 7.69 (3H, m, 3,4,5H Bz), 8.06 (2H, d, *J* = 7 Hz, 2,6 H Bz), 8.70 (1H, s, 8-H), 8.77 (1H, s, 2-H), 11.21 (1H, s, ex, NH); $^{13}$C-NMR (50 MHz, DMSO-$d_6$): δ = 61.1 ($CH_2$6'), 69.9 (CH4'), 71.3 (CH2'), 77.3 (CH3'), 80.3 (CH5'), 83.2 (CH1'), 125.7 (5-C), 128.7 (2,3,5,6CH Bz), 132.7 (4CH Bz), 133.6 (1-C Bz), 143.8 (2-CH), 150.5 (4-C), 152.0 (8 CH), 153.0 (6-C), 165.9 (C=O); MS (ESI; m/z (%)): 105 (40) [Bz]+, 240 (76), 262 (70) 402 (11) [M+H]+, 424 (100) [M+Na]+, 446 (56) [M-H+2Na]+; Exact Mass: $C_{18}H_{20}N_5O_6$: Calc.: 402.1413; Found: 402.1418.
N[4]-Benzoyl-1-(β-D-glucopyranosyl)cytosine: Yield: 1.04 g (90 %); $R_f$ (ethyl acetate) 0.52, mp: 189-191°C ($CH_2Cl_2$, lit: 191-192°C)[18]

INTRODUCTION OF MONOMETHOXYTRITYL IN THE 6' POSITION: GENERAL PROCEDURE:

The mixture of nucleoside A orC (3.5 mmol), respectively and MMTCl (2.16 g, 7.0 mmol) in pyridine (20 mL) and triethylamine (5 mL) was stirred in an inert atmosphere at ambient temperature for 18 h. After removing the solvent in vacuo the resultant oil was coevaporated with toluene (4 x 2 mL). The resulted foam was purified by column chromatography. (silica, 15 x 3 cm, $CH_2Cl_2$/MeOH = 9 : 1).
6'-*O*-Monomethoxytrityl-N[6]-benzoyl-9-(β-D-glucopyranosyl)adenine: Yield: 1.6 g (70 %); $R_f$ ($CH_2Cl_2$/MeOH = 9 : 1) 0.27, mp: 148 - 152°C; $^1$H-NMR (200 MHz, DMSO-$d_6$): δ = 2.88 - 3.39 (6H, m, 2 x H6', H5', H3', H4', H2'), 3.70 (3H, s, $CH_3O$), 5.17, 5.40, 5.53 (3H, ex, 4 x OH), 5.67 (1H, d, *J* = 9 Hz, H1'), 6.74 (2H, d , *J* = 8.4 Hz, AA'BB'), 7.24 - 7.59 (15H, m, CH MMT, Bz,), 8.06 (2H, d, *J* = 7 Hz, 2,6 H Bz), 8.72 (1H, s, 8-H), 8.80 (1H, s, 2-H), 11.25 (1H, s, ex,

NH); $^{13}$C-NMR (50 MHz, DMSO-$d_6$): δ = 55.1 (CH$_3$O), 63.9 (CH$_2$6'), 70.1 (CH4'), 71.2 (CH2'), 77.3 (CH3'), 78.2 (CH5'), 83.4 (CH1'), 85.6 (C MMT), 96.9 (5-C), 113.2 (AA'BB', MMT), 125.8 (5-C), 126.9- 130.3 (C Ar MMT + Bz), 132.6 (4CH Bz), 133.6 (1C Bz), 135.4 (AA'BB' MMT), 143.6 (2-CH), 144.7 (2 x 1C MMT), 150.3 (4-C), 151.9 (8-CH), 153.0 (6-C), 158.3 (4C AA'BB' MMT), 165.9 (C=O Bz); MS (ESI; m/z (%)): 105 (22), [Bz]$^+$, 240 (37), 273 (100), [MMT]$^+$, 696 (30) [M + Na]$^+$, 718 (8) [M - H + 2Na]$^+$; Exact Mass: C$_{38}$H$_{36}$N$_5$O$_7$: Calc.: 674.2614; Found: 674.2618.

6'-O-Monomethoxytrityl-N$^4$-benzoyl-1-(β-D-glucopyranosyl)cytosine: Yield: 2.27 g (83%); R$_f$ (CH$_2$Cl$_2$/MeOH = 9 : 1) 0.25, mp: 150 - 154 C; $^1$H-NMR (200 MHz, DMSO-$d_6$): δ = 2.92 - 3.72 (6H, m, 2 x H6', H5', H3', H4', H2'), 3.68 (3H, s, CH$_3$O), 5.10, 5.34, 5.43 (3H, ex, 4 x OH), 5.66 (1H, d, J = 9 Hz, H1'), 6.84 (2H, d , J = 8.4 Hz, AA'BB'), 7.24 - 7.63 (16H, m, CH MMT, Bz, 5-H), 8.01 (2H, d, J = 7 Hz, 2,6 H Bz), 8.21 (1H, d, J = 7.6 Hz, 6-H), 11.28 (1H, s, ex, NH); $^{13}$C-NMR (50 MHz, DMSO-$d_6$): δ = 55.1 (CH$_3$O), 64.1 (CH$_2$6'), 70.1 (CH4'), 71.8 (CH2'), 77.2 (CH3'), 78.5 (CH5'), 83.5 (CH1'), 85.6 (C MMT), 96.9 (5-C), 113.3 (AA'BB', MMT), 126.9 - 130.3 (C Ar MMT + Bz), 133.0 (4CH Bz), 133.4 (1C Bz), 135.4 (1C AA'BB'), 144.5 (2 x 1C MMT), 146.3 (6-CH), 155.0 (2-C=O), 158.3 (4C AA'BB' MMT), 163.2 (C=O Bz); MS (ESI; m/z (%)): 105 (43), [Bz]$^+$, 238 (40), 273 (100), [MMT]$^+$, 672 (8) [M+Na]$^+$; Exact Mass: C$_{37}$H$_{37}$N$_3$O$_8$: Calc.: 651.2580; Found:. 651.2588.

<u>OXIDATIVE RING OPENING OF A,C: GENERAL PROCEDURE:</u>

To a solution of 6'-O-MMT nucleoside A,C respectively(1 mmol) in absolute dichloromethane (10 mL), Pb (OAc)$_4$ (1.33 g, 3 mmol) is added under nitrogen at ambient temperature. The solution gets warm and turns yellow. After 2 h a colorless precipitate of Pb(OAc)$_2$ is formed. Stirring is continued over night. After removing the solvent on a rotavapor the residue is purified by column chromatography (silica, 10 x 2 cm, CH$_2$Cl$_2$/MeOH = 95 : 5). MS verified the structure of the resultant colorless foams because of the complicated equilibrium observed in the NMR.

5'-O-Monomethoxytrityl-N$^6$-benzoyladenosine dialdehyde: Yield: 0.56 g (88 %); R$_f$ (CH$_2$Cl$_2$/MeOH = 95 : 5) 0.54, MS (LSIMS; Thgly; m/z (%)):273 (100) [MMT]$^+$, 640 (2) [M + Na]$^+$, 662 (1) [M - H + 2 Na]$^+$.

5'-O-Monomethoxytrityl-N$^4$-benzoylcytidine dialdehyde: Yield: 0.57 g (92 %); R$_f$ (CH$_2$Cl$_2$/MeOH = 95 : 5) 0.49, MS (LSIMS; Thgly; m/z (%)):273 (100) [MMT]$^+$, 664 (1) [M + Na]$^+$, 686 (2.8) [M - H + 2 Na]$^+$.

<u>REDUCTIVE RING CLOSURE OF A,C: GENERAL PROCEDURE:</u>

A,C-dialdehyde (1 mmol), respectively, was dissolved in absolute dioxane (8 mL) under a nitrogen atmosphere. Tri-n-butyltin hydride (0.43 g, 0.4 mL, 1.25 mmol) is added via a septum. The reaction mixture is heated to 90°C. Via the septum a solution of AIBN (0.02 g, 0.12 mmol) in dioxane (2 mL) is added. Foam is formed, after stirring for 30 min at 90°C the reaction mixture was checked with TLC (CH$_2$Cl$_2$/MeOH = 95 : 5). If unreacted aldehyde is monitored on TLC, additional AIBN (0.01g, 0.06 mmol) and tri-n-butyltin hydride (0.11g, 0.1 mL, 0.31 mmol) are added and stirring at 90°C is continued for another 60 min. After evaporating the solvent under reduced pressure the residue is purified by column chromatography (silica, 20 x 3 cm, CH$_2$Cl$_2$ (100 mL) → CH$_2$Cl$_2$/MeOH = 95 : 5).

5'-Monomethoxytrityl-N$^6$-benzoyladenosine: Yield: 0.34g (53%); R$_f$ (CH$_2$Cl$_2$/MeOH = 9 : 1 ) 0.49, mp: 122-125 C (CH$_2$Cl$_2$), (Lit: 120 - 125 C)[19].

5'-Monomethoxytrityl-N$^4$-benzoylcytidine: Yield: 0.31 g (54 %); R$_f$ (CH$_2$Cl$_2$/MeOH = 9 : 1 ) 0.38, mp: 140-146 C (CH$_2$Cl$_2$), (Lit: 140-146 C)[19].

<u>PREPARATION OF [1',2',3',4',5'-$^{13}$C$_5$]-(2',3',5'-TRI-O-ACETYL)URIDINE FROM [1',2',3',4',5'-$^{13}$C$_5$]-5'-O-MONOMETHOXYTRITYL-URIDINE:</u>

A mixture of [1',2',3',4',5'-$^{13}$C$_6$]-5'-O-monomethoxytrityl-uridine (0.5 g, 1 mmol) in 80 % acetic acid (5 mL) was stirred at room temperature for 30 min. After addition of 50 mL water, the precipitate was filtered off, the solution was evaporated under reduced pressure. The resulted crude product was treated with acetic anhydride (2.5 mL) without further purification. After heating under reflux for 2 h, the reaction mixture was cooled to ambient temperature and neutralized with aqueous NaHCO$_3$. The mixture was extracted with CH$_2$Cl$_2$ (3 x 10 mL), the organic layer was dried over Na$_2$SO$_4$ and removed on the rotavapor. Further purification was achieved by column chromatography (silica, 10 x 3 cm, CH$_2$Cl$_2$/MeOH = 95 :5).

[1',2',3',4',5'-$^{13}$C$_6$]-(2',3',5'-Triacetyl)uridine: 0.28 g, (79 %); R$_f$ (CH$_2$Cl$_2$/MeOH =9 : 1 ): 0.65; mp: 128 -130°C (MeOH); $^1$H-NMR (500 MHz, CDCl$_3$): δ = 2.06, 2.10, 2.19 ( 3 x 3H, 3 x Ac), 4.31 (3H, m, J$^{CH}$ = 162 Hz, 2x H5', H2'), 5.33 (2H, m, J$^{CH}$ = 162 Hz, H3', H4'), 5.76 (1H, d, J = 8 Hz, H-5), 6.01 (1H, J$^{CH}$ = 160 Hz, J$^{HH}$ = 5 Hz, H1'), 7.40 (1H, J = 8 Hz, 6-H), 9.82 (1H, s, br, ex, NH); $^{13}$C-NMR (50 MHz, CDCl$_3$): δ = 20.2, 20.3, 20.5 (3 x CH$_3$ Ac), 63.0 (d, J= 43 Hz, CH5'), 70.0 (t, J= 43 Hz, CH2'), 72.5 (t, J= 43 Hz, CH3'), 79.8 (t, J= 43 Hz, CH4'), 87.4 (d, J = 45 Hz, CH1'), 103.3 (s, 5-CH), 139.4 (s, 6-CH), 150.3 (s, 2-C), 163.1 (s, 4-C), 169.6, 169.7, 170.2 (3 x C=O Ac); MS (ESI; m/z (%)):139 (73), 259 (74), 376 (8) [M + H]$^+$, 398 (100) [M + Na]$^+$, 420 (36) [M - H + 2 Na]$^+$; Exact Mass: C$_{10}$C$_5^*$H$_{18}$N$_2$O$_9$Na: Calc.:

398.1078; Found:. 398.1086.

TRANSGLYCOSYLATION OF U→G:

To the suspension of tri-O-acetyluridine (0.65 g, 1.77 mmol) and $N^2$-isobutyrylguanine (2.35 g, 10.62 mmol) in dichloroethane (40 mL) BSA (12 mL, 50 mmol) was added. The mixture was heated under nitrogen to result in a clear, colorless solution. After addition of TMSOTf (2.4 mL, 13 mmol) under nitrogen the reaction mixture was heated under reflux for 10 h. After cooling to ambient temperature, brine (150 mL) was added. The precipitate was filtered off and washed with $CH_2Cl_2$ (5 x 10 mL). After extracting the aqueous layer with $CH_2Cl_2$ (30 mL) the combined organic layers were dried over $Na_2SO_4$. After evaporating the solvent the resulted oil was purified by column chromatography (silica, 24 x 3 cm, $CH_2Cl_2$/MeOH = 95 : 5). (2'.3',5'-Tri-O-acetyl)-$N^2$-isobutyrylguanosine: Yield 0.73 g ( 86 %); $R_f$ ($CH_2Cl_2$/MeOH = 95 : 5) 0.39; mp: 125 - 130°C[20].

References

[1.] Kainosho, M. (1997) *Nature Struct. Biol. NMR Suppl.,* 858-861.

[2.] Milligan, J.F., Groebe, D.R., Witherell, G.W., Uhlenbeck, O.C. (1987) *Nucleic Acids. Res.,* **15**, 8783-8799.

Wyatt, J.R., Chastain, M., Puglisi, J.D. (1991) *BioTechniques,* **11**, 764-769.

Nikonowicz, E.P., Sirr, A., Legault, P., Jucker, F.M., Baer, L.M., Pardi, A. (1992) *Nucleic Acids Res.,* **20**, 4507-4513.

Batey, R.T., Inada, M., Kujawinski, E., Puglisi, J.D., Williamson, J.R. (1992) *Nucleic Acids Res.,* **20**, 4515-4523.

Michnicka, M.J., Harper, J.W., King, G.C. (1993) *Biochemistry,* **32**, 395-400.

Nikonowicz, E.P., Pardi, A. (1993) *J. Mol. Biol.,* **232**, 1141-1156.

Hines, J.V., Landry, S.M., Varani, G., Tinoco Jr., I., *(1994) J. Am. Chem. Soc.,* **116**, 5823-5831.

[3.] Parkin, D.W., Leung, H.B., Schramm, V.L. (1984) *J. Biol. Chem.,* **259**, 9411-9417.

Parkin, D.W., Schramm, V.L. (1987) *Biochemistry,* **26**, 913-920.

Gilles A.-M., Cristea, I., Palibroda, N., Hilden, I., Jensen, K.F., Sarfati, R.S., Namane, A., Ughetto-Monfrin, J., Bârzu, O. (1995) *Anal. Biochem.,* **232**, 197-203.

Tolbert, T.J., Williamson, J.R. *(1997) J. Am. Chem. Soc.,* **119**, 12100-12108.

[4.] Zimmer, D.P., Crothers, D.M. (1995) *Proc. Natl. Acad. Sci USA,* **92**, 3091-3095.

[5.] G., Chazin, W.J. (1998) *J. Am. Chem. Soc.,* **120**, 607-608.

[6.] Masse, J.E., Bortmann, P., Dieckmann, T., Feigon, J. (1998) *Nucleic Acids Res.,* **26**, 2618-2624.

[7.] Louis, J.M., Martin, R.G., Clore, G.M., Gronenborn, A.M. *(1998) J. Biol. Chem.,* **273**, 2374-2378. [8.] Tate, S.-i., Ono, A., Kainosho, M. (1994) *J. Am. Chem. Soc.,* **116**, 5977-5978.

Tate, S.-i., Ono, A., Kainosho, M. (1995) *J. Magn. Res. serB,* **106**, 89-91.

Szyperski, T., Ono, A., Fernandez, C., Iwai, H., Tate, S.-i., Wüthrich, K., Kainosho, M. (1997) *J. Am. Chem. Soc.,* 9901-9902.

[9.] Kline, P.C., Serianni, A.S. (1990) *J. Am. Chem. Soc.,* **112**, 7373-7381.

Lancelot, G., Chanteloup, L., Beau, J.-M., Thuong, N.T. *(1993) J. Am. Chem. Soc.,* **115**, 1599-1600. Bornet, O., Lancelot, G., Chanteloup, L., Thuong, N.T., Beau, J.-M. (1994) *J. Biomol. NMR,* **4**, 575-580. Ono, A., Tate, S.-i., Ishido, Y., Kainosho, M. 1994, J. *Biomol. NMR,* **4**, 581-586.

Tolbert, T.J., Williamson, J.R. (1996) *J. Am. Chem. Soc.,* **118**, 7929-7940

[10.] Milecki, J., Zamaratski,E., Maltseva,T.V., Földesi,A., Adamiak,R.W., Chattopadhyaya, J. (1999) *Tetrahedron,* **55**, 6603-6622

[11.] Quant, S., Wechselberger, R.W., Wolter, M.A., Wörner, K.-H., Schell, P., Engels, J.W., Griesinger, C., Schwalbe, H. (1994) *Tetrahedron Lett.*, **35**, 6649-6652.

[12.] Agrofoglio, L.A., Jacquinet, J.-C., Lancelot, G. (1997) *Tetrahedron Lett.,* **38**, 1411-1412.

[13.] Vorbruggen, H. and Bennua, B. (1981) *Chem. Ber.* ,**114**, 1234.

[14.] Howarth, O., Jones, A.S., Walker, R.T., Wyatt, P.G. (1984) J. *Chem. Soc. Perkin Trans II,* 261-265.

[15.] Yamaoka, N., Asu, K., Matsuda, K. (1965) J. *Org. Chem.,* **30**, 149-152.

[16.] Lerner, L.M. and Kohn, P. (1965) *J. Med. Chem.*, **7**, 655-658.

[17.] Hilbert, C.W. and Jansen, E.F. (1936) *J. Am. Chem. Soc.*, **58**, 60-62.

[18.] Lichterthaler, F., Ueno,T., Voss, P. (1974) *Bull. Chem. Soc. Jpn.* **47**, 2304-2307.

[19.] Flockerzi, D., Silber, G., Charubala, R., Schlosser, W., Varma, R.S., Creegan, F., Pfleiderer, W., (1981) *Liebigs Ann. Chem.* **9**, 1568 - 1585.

[20.] Robins, M.J., Zou, R., Guo, Z., Wnuk, S.F., (1996) J. *Org. Chem.,* **61**, 9207-9212.

**Claims**

1. Method for the chemical preparation of labeled nucleosides via an oxidative-ring-opening step and a reductive-ring-closure step starting from a labeled D-glucose nucleoside resulting in the corresponding D-ribose-nucleoside by removing the carbon atom 3 on the glucose.

2. Method according to claim 1, wherein the oxidative-ring-opening is performed using $Pb(OAc)_4$.

3. Method according to claim 1 or 2, wherein the reductive-ring-closure is obtained using $Bu_3SnH$.

4. Method according to claim 1 or 2, wherein the reductive-ring-closure is obtained using $SmI_2$.

5. Method according to claim 1, 2 or 3, wherein the reductive-ring-closure is obtained using AIBN.

6. Method according to any of the previous claims 1-5, starting from labeled [$^{13}C_6$]-D-glucose comprising the steps of:

    1. a protection of the sugar ring hydroxyl groups,
    2. a sugar-base condensation, whereby the base is a purine or a pyrimidine,
    3. a deprotection of the sugar ring hydroxyl groups,
    4. an oxidative-ring-opening and removal of the carbon atom 3, and
    5. a reductive-ring-closure resulting in labeled D-ribose-nucleoside.

7. Method according to claim 6, wherein step 3 is followed by a protection step of the primairy OH-group,

8. Method according to claim 7, wherein the protection step comprises a mono-$C_1$-$C_6$-alkyloxytritylation or a bis-$C_1$-$C_6$-alkyloxytritylation of the primary hydroxyl function in the 6'-O-position of glucose.

9. Method according to claim 8, wherein the mono-$C_1$-$C_6$-alkyloxy is mono-methyloxy or wherein the bis-$C_1$-$C_6$-alkyloxy is di-methyloxy.

10. Method according to any of the claims 6-9, wherein step 3 is followed by a protection step of the base moiety.

11. Method according to any of the previous claims 1-10, wherein if the nucleoside is uracil a transglycosylation step is performed resulting in guanine.

12. Method according to any of the previous claims 1-11, further comprising the functionalization of the obtained ribonucleosides into protected ribonucleoside phosphoramidites, being preferably 2'-O-silyl protected ribonucleoside phosphoramidites.

13. Method according to any of the previous claims 1-12, further comprising the deoxygation of the obtained ribonucleosides into deoxyribonucleosides.

14. Method according to claim 13, further comprising the functionalization of the obtained deoxyribonucleosides into protected deoxyribonucleoside phosphoramidites.

15. Compound obtainable via the method according to any of the previous claims 1-10 having the formula selected from [$^{13}C,^{15}N$] labeled 6'-O-mono- or 6'-O-bis-$C_1$-$C_6$-alkyloxytrityl-D-glycopyranosyl nucleosides, or [$^{13}C,^{15}N$] labeled tetra-O-acetyl-D-glycopyranosyl nucleosides or [$^{13}C,^{15}N$] labeled glycopyranosyl nucleosides.

16. Compound according to claim 15, wherein the mono-$C_1$-$C_6$-alkyloxy is mono-methyloxy or wherein the bis-$C_1$-$C_6$-alkyloxy is di-methyloxy.

17. Compound obtainable via the method according to any of the previous claims 1-14 having the formula of [$^{13}C,^{15}N$] labeled 5'-O-mono- or 5'-O-bis-$C_1$-$C_6$-alkyloxytrityl-ribonucleosides.

18. Compound according to claim 17, wherein alkyloxy is methyloxy or ethyloxy.

19. Compound according to claim 17 or 18, wherein the ribonucleosides are chosen from adenosine, guanosine, cytidine, uridine and 5-methyluridine.

20. Compound obtainable via the method according to any of the previous claims 1-14, having the formula of [$^{13}$C,$^{15}$N] labeled 5'-O-mono- or 5'-O-bis-$C_1$-$C_6$-alkyloxytrityldeoxyribonucleosides.

21. Compound according to claim 20, wherein alkyloxy is methyloxy or ethyloxy.

22. Compound according to claim 20 or 21, wherein the deoxyribonucleosides are chosen from deoxyadenosine, deoxyguanosine, deoxycytidine and thymidine.

23. Compound according to any of the previous claims 15-22, having the formula of:

[1',2',3',4',5',6'-$^{13}$C$_6$]-[2',3',4',6'-tetra-*O*-acetyl-β-D-glucopyranosyl)uracil

[1',2',3',4',5',6'-$^{13}$C$_6$]-[2',3',4',6'-tetra-*O*-acetyl-β-D-glucopyranosyl)-[6-$^{13}$C, 1,3-$^{15}$N$_2$]-uracil

[1',2',3',4',5',6'-$^{13}$C$_6$]-(β-D-glucopyranosyl)uracil

[1',2',3',4',5',6'-$^{13}$C$_6$]-(β-D-glucopyranosyl)-(6-$^{13}$C, 1,3-$^{15}$N$_2$)-uracil

[1',2',3',4',5',6'-$^{13}$C$_6$]-6'-*O*-monomethoxytrityl-(β-D-glucopyranosyl)uracil

[1',2',3',4',5',6'-$^{13}$C$_6$]-6'-*O*-monomethoxytrityl-(β-D-glucopyranosyl)-[6-$^{13}$C, 1,3-$^{15}$N$_2$]-uracil

[1',2',3',4',5',6'-$^{13}$C$_6$](2',3',4',6'-tetra-*O*-acetyl-β-D-glucopyranosyl)-N$^4$-benzoylcytosine

[1',2',3',4',5',6'-$^{13}$C$_6$](2',3',4',6'-tetra-*O*-acetyl-β-D-glucopyranosyl)-[6-$^{13}$C, 1,3,NH$_2$-$^{15}$N$_3$]-N$^4$-benzoylcytosine

[1',2',3',4',5',6'-$^{13}$C$_6$]-(β-D-glucopyranosyl)-cytosine

[1',2',3',4',5',6'-$^{13}$C$_6$]-(β-D-glucopyranosyl)-[6-$^{13}$C, 1,3,NH$_2$-$^{15}$N$_3$]-cytosine

[1',2',3',4',5',6'-$^{13}$C$_6$]-(β-D-glucopyranosyl)-N$^4$-benzoylcytosine

[1',2',3',4',5',6'-$^{13}$C$_6$]-(β-D-glucopyranosyl)-[6-$^{13}$C, 1, 3,NH$_2$-$^{15}$N$_3$]-N$^4$-benzoylcytosine

[1',2',3',4',5',6'-$^{13}$C$_6$]-(6'-*O*-monomethoxytrityl-β-D-glucopyranosyl)-N$^4$-benzoylcytosine

[1',2',3',4',5',6'-$^{13}$C$_6$]-(6'-*O*-monomethoxytrityl-β-D-glucopyranosyl)-[6-$^{13}$C,    1,3,NH$_2$-$^{15}$N$_3$]-N$^4$-benzoylcytosine

[1',2',3',4',5'-$^{13}$C$_5$]-(2',3',5'-tri-*O*-acetyl)-uridine

[1',2',3',4',5',6'-$^{13}$C$_6$] (2',3',4',6'-tetra-*O*-acetyl-β-D-glucopyranosyl)-N$^6$-benzoyladenine

[1',2',3',4',5',6'-$^{13}$C$_6$] (2',3',4',6'-tetra-*O*-acetyl-β-D-glucopyranosyl)-[8-$^{13}$C, 9,NH$_2$-$^{15}$N$_2$]-N$^6$-benzoyladenine

[1',2',3',4',5',6'-$^{13}$C$_6$]-(β-D-glucopyranosyl)-adenine

[1',2',3',4',5',6'-$^{13}$C$_6$]-(β-D-glucopyranosyl)-[8-$^{13}$C, 9,NH$_2$-$^{15}$N$_2$]-adenine

[1',2',3',4',5',6'-$^{13}$C$_6$]-(β-D-glucopyranosyl)-N$^6$-benzoyladenine

[1',2',3',4',5',6'-$^{13}$C$_6$]-(β-D-glucopyranosyl)-[8-$^{13}$C, 9,NH$_2$-$^{15}$N$_2$]-N$^6$-benzoyladenine

[1',2',3',4',5',6'-$^{13}$C$_6$]-(6'-*O*-monomethoxytrityl-β-D-glucopyranosyl)-N$^6$-benzoyladenine

[1',2',3',4',5',6'-$^{13}$C$_6$]-(6'-*O*-monomethoxytrityl-β-D-glucopyranosyl)-[8-$^{13}$C, 9,NH$_2$-$^{15}$N$_2$]-N$^6$-benzoyladenine.

24. Compound according to any of the claims 15-23 obtained via the method according to any of the previous claims 1-14.

25. Use of a compound according to any of the previous claims 15-24, in mass spectrometry and diagnostics.

26. Use of a compound according to any of the previous claims 15-25, for NMR applications.

27. Use of a compound obtainable via the method of claim 12 or claim 14 for oligonucleotide synthesis.

28. Compounds obtained by the method according to claim 1-14.

D-glucopyranose                    D-glucofuranose

**Figure 1**

**Figure 2** i: Ac$_2$O, pyr, 0 °C, 97%; ii: [6-$^{13}$C, 1,3-$^{15}$N$_2$]-uracil, ClCH$_2$CH$_2$Cl, BSA, TMSOTf, $\Delta$, 87.5 %; iii: NH$_3$, MeOH, 98 %; iv: MMTrCl, pyr, Et$_3$N, 92%; v: Pb( OAc)$_4$, CH$_2$Cl$_2$, 90 %; vi: Bu$_3$SnH, AIBN, dioxane, 62%. The isotopically labeled C-atoms are marked as • and the N-atoms are indicated in bold.

**9** B: N$^6$-benzoyladenine      **10** B: N$^6$-benzoyladenine

**Figure 3** i: Pb(OAc)$_4$, CH$_2$Cl$_2$; ii: Bu$_3$SnH, AIBN, dioxane.

## PARTIAL EUROPEAN SEARCH REPORT

Application Number

which under Rule 45 of the European Patent Convention EP 00 87 0188
shall be considered, for the purposes of subsequent
proceedings, as the European search report

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| D,Y | ONO, AKIRA ET AL: "Preparation and heteronuclear 2D NMR spectroscopy of a DNA dodecamer containing a thymidine residue with a uniformly 13C-labeled deoxyribose ring" J. BIOMOL. NMR (1994), 4(4), 581-6 , XP000982201 * abstract * | 1-28 | A61K51/04 |
| D,Y | QUANT, S. ET AL: "Chemical synthesis of 13C-labeled monomers for the solid-phase and template controlled enzymic synthesis of DNA and RNA oligomers" TETRAHEDRON LETT. (1994), 35(36), 6649-52 , XP002160278 See scheme 1, p. 6650, step 1, | 1-28 | |

-/--

TECHNICAL FIELDS
SEARCHED (Int.Cl.7)

A61K

### INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched .

Reason for the limitation of the search

see sheet C

| Place of search | Date of completion of the search | Examiner |
|---|---|---|
| THE HAGUE | 14 February 2001 | Berte, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03 82 (P04C07)

European Patent
Office

**INCOMPLETE SEARCH
SHEET C**

Application Number

EP 00 87 0188

Reason for the limitation of the search:

Present claims 1-28 relate to an extremely large number of possible
compounds and their methods of preparation. Support within the meaning of
Article 84 EPC and/or disclosure within the meaning of Article 83 EPC is
to be found, however, for only a very small proportion of the compounds
and methods claimed. In the present case, the claims so lack support, and
the application so lacks disclosure, that a meaningful search over the
whole of the claimed scope is impossible. Consequently, the search has
been carried out for those parts of the claims which appear to be
supported and disclosed, namely those parts relating to the compounds and
methods covered by the search e.g those compounds or methods prepared in
the examples and closely related homologous compounds  and the ones cited
in the claims.

European Patent
Office

**PARTIAL EUROPEAN SEARCH REPORT**

Application Number

EP 00 87 0188

| | DOCUMENTS CONSIDERED TO BE RELEVANT | | CLASSIFICATION OF THE APPLICATION (Int.Cl.7) |
|---|---|---|---|
| Category | Citation of document with indication, where appropriate, of relevant passages | Relevant to claim | |
| D,A | MILECKI, JAN ET AL: "The first example of sequence-specific non-uniformly 13C5 labeled RNA: synthesis of the 29mer HIV-1 TAR RNA with 13C relaxation window" TETRAHEDRON (1999), 55(21), 6603-6622 , XP002160122 Scheme 1, p. 6605, | 1-28 | |
| A | ONO, AKIRA MEI ET AL: "Systematic synthesis of specifically 13C/2H-labeled nucleosides from 'u1-13C6!-D-glucose" TETRAHEDRON LETT. (1998), 39(18), 2793-2796 , XP002160123 * figure 1 * | 1-28 | |
| X | AGROFOGLIO, LUIGI A. ET AL: "A multigram, stereoselective synthesis of D-' 13C5 !ribose from D-' 13C6 !glucose and its conversion into ' 13C5 !nucleosides" TETRAHEDRON LETT. (1997), 38(8), 1411-1412 , XP002160124 | 1,2 | |
| Y | * abstract * | 1-28 | |

TECHNICAL FIELDS SEARCHED (Int.Cl.7)

EPO FORM 1503 03.82 (P04C10)